# EUROPÄISCHE PATENTSCHRIFT

(11) **EP 3 922 986 B1**
(45) Veröffentlichungstag und Bekanntmachung des Hinweises auf die Patenterteilung: **24.07.2024**
(21) Anmeldenummer: 20179370.0
(22) Anmeldetag: 10.06.2020
(51) Int. Cl.: G01N 23/06, G01N 33/28

(54) **VERFAHREN ZUM MESSEN VON ZÄHLRATEN ODER VON DEN ZÄHLRATEN ABHÄNGIGEN MESSGRÖSSEN UND VORRICHTUNG ZUM MESSEN VON ZÄHLRATEN ODER VON DEN ZÄHLRATEN ABHÄNGIGEN MESSGRÖSSEN**
METHOD FOR MEASURING COUNTING RATES OR MEASURED VALUES DEPENDENT ON THE COUNTING RATES, AND DEVICE FOR MEASURING COUNTING RATES OR MEASUREMENT VARIABLES DEPENDENT ON THE COUNTING RATES
PROCÉDÉ DE MESURE DE TAUX DE COMPTAGE OU DE GRANDEURS MESURÉES EN FONCTION DES TAUX DE COMPTAGE ET DISPOSITIF DE MESURE DE TAUX DE COMPTAGE OU DE GRANDEURS MESURÉES EN FONCTION DES TAUX DE COMPTAGE

(43) Veröffentlichungstag der Anmeldung: 15.12.2021
(73) Patentinhaber: Berthold Technologies GmbH & Co. KG, 75323 Bad Wildbad (DE)
(72) Erfinder: Müller, Steffen, Dr., 75173 Pforzheim (DE)
(74) Vertreter: Patentanwälte Ruff, Wilhelm, Beier, Dauster & Partner mbB

(56) Entgegenhaltungen:
- EP-A1- 3 264 078
- WO-A2-03/052396
- GB-A- 2 569 322
- US-A1- 2019 078 916

## Beschreibung

Die Erfindung betrifft ein Verfahren sowie eine Vorrichtung zum Messen von Zählraten oder von den Zählraten abhängigen Messgrößen, insbesondere für die Prozessmesstechnik.

Derartige Verfahren und Vorrichtungen können beispielsweise in Multi-Level-Messungen verwendet werden, mit welchen Füllstände mehrerer Flüssigkeiten in einem Tank radiometrisch gemessen bzw. erfasst werden können. Eine häufige Anwendung hierfür ist zum Beispiel ein Öl-Wasser-Separator in der Ölindustrie. In den Separator wird beispielsweise ein Gemisch aus Öl, Wasser, Sand und Gas gegeben, der Separator soll nun das Gemisch über die Gravitation trennen, so dass die einzelnen Flüssigkeiten getrennt abgeleitet werden können. Je präziser die einzelnen Füllstände sind, desto effizienter und kostensparender kann der Separator betrieben werden.

Eine solche Messaufgabe wird bereits heute radiometrisch erfüllt, wobei sich typischerweise Strahler in einem Tauchrohr im Separator befinden und die Detektoren außerhalb angebracht sind. Jeder Detektor ist dabei typischerweise so kalibriert, dass er die Dichte des Materials auf Detektorhöhe misst. Dies wird z.B. dadurch realisiert, dass jedem Detektor ein Strahler auf gleicher Höhe zugeordnet ist. Aus dem Dichteprofil kann dann auf die einzelnen Schichten zurückgeschlossen werden.

Im Übrigen offenbart die US 2019/078916 A1 ein Verfahren zum Ableiten von Einfallszählraten von elektromagnetischer Energie an einem Detektor. In einer Ausführungsform umfasst das Verfahren ein Übertragen elektromagnetischer Strahlung durch ein Fluid und ein Empfangen eines Teils der elektromagnetischen Strahlung an einem Detektor. Das Verfahren umfasst auch ein Messen des Energiespektrums des vom Detektor empfangenen Teils der elektromagnetischen Strahlung und ein Verwenden des gemessenen Energiespektrums und eines physikalischen Modells der Detektorreaktion auf elektromagnetische Strahlung, um Einfallszählraten für diskrete Energieniveaus des Teils der elektromagnetischen Strahlung empfangen vom Detektor abzuleiten. Zusätzliche Systeme, Vorrichtungen und Verfahren werden auch offenbart.

Des Weiteren offenbart die GB 2 568 322 A ein Probennahmemodul zum Anbringen in einer Rohrleitung mit einem Mehrphasen-Durchflussmesser und zum Aufnehmen von Mehrphasenfluid aus der Rohrleitung, wobei das Probennahmemodul umfasst: eine Trennkammer zum Aufnehmen und Abtrennen eines Probenvolumens von Fluid aus dem Mehrphasenfluid, wobei die Trennkammer in Verwendung eine vertikale Ausdehnung aufweist; ein unteres Ventil zum Öffnen und Schließen eines Fluidwegs zwischen einem unteren Ende der Trennkammer und der Rohrleitung; ein oberes Ventil zum Öffnen und Schließen eines Fluidwegs zwischen einem oberen Ende der Trennkammer und der Rohrleitung; einen unteren Sensor zum Messen von Fluideigenschaften des Fluids in einem unteren Teil der Trennkammer; und einen oberen Sensor zum Messen von Fluideigenschaften des Fluids in einem oberen Teil der Trennkammer. Die Sensoren können einen Gamma-Densitometer, Permittivitätssensor, Leitfähigkeitssensor, Temperatursensor, Drucksensor umfassen. In Zeiten, in denen das Probennahmemodul nicht verwendet wird, kann der Trennkammer ein Hydratblocker zugeführt werden, bevor sie geschlossen wird. Eine Heizvorrichtung kann verwendet werden, um die Trennkammer zu heizen. Es wird auch ein Verfahren zum Verwenden des Probennahmemoduls zum Erhalten von Fluideigenschaftsmessungen als Eingangsparameter für einen Mehrphasen-Durchflussmesser offenbart.

### AUFGABE UND LÖSUNG

Mit der Erfindung wurde jedoch erkannt, dass es insbesondere bei runden Separatorgeometrien unumgänglich ist, dass ein Übersprechen der verschiedenen Punktstrahler in die anderen Detektoren stattfindet. Dieses Übersprechen liefert einen großen Beitrag zum Gesamtfehler des Systems, da die Dichten räumlich nicht mehr scharf bestimmt werden können. Kollimatoren können diesbezüglich zwar eingesetzt werden, lösen das Problem jedoch typischerweise nicht vollständig. Das Übersprechen ist auch nicht durch eine gute Kalibrierung zu eliminieren, da der Anteil des Übersprechens abhängig von den Flüssigkeitsständen oberhalb und unterhalb des Detektors ist.

Es ist deshalb eine Aufgabe der Erfindung, ein Verfahren zum Messen von Zählraten oder von den Zählraten abhängigen Messgrößen für ein Bestimmen eines Dichteprofils vorzusehen, welches genauere Ergebnisse liefert. Es ist des Weiteren eine Aufgabe der Erfindung, eine zugehörige Vorrichtung vorzusehen.

Dies wird erfindungsgemäß durch ein Verfahren gemäß Anspruch 1 und eine Vorrichtung gemäß Anspruch 12 erreicht. Vorteilhafte Ausgestaltungen sind in den Unteransprüchen beansprucht.

Die Erfindung betrifft ein, insbesondere automatisches, Verfahren zum, insbesondere automatischen, Messen von Zählraten oder von, insbesondere zu, den Zählraten abhängigen, insbesondere proportionalen, Messgrößen für ein Bestimmen eines Dichteprofils von mindestens zwei, insbesondere mindestens drei, Stoffen mit verschiedenen Dichten, insbesondere verschiedenen Werten der Dichten, welche innerhalb eines Behälters angeordnet sind, und zwar mittels einer Mehrzahl von, insbesondere gammaenergiesensitiven, Detektoren. Das Verfahren weist die folgenden Schritte auf:
a) jeweils, insbesondere automatisches und/oder gammaenergiesensitives, Erfassen von jeweiligen Gammastrahlen, welche jeweils mindestens einen der Stoffe mindestens teilweise durchdrungen haben, mittels der Detektoren, und
b) jeweils, insbesondere automatisches, Erzeugen einer jeweiligen Zählrate, insbesondere eines Werts der Zählrate, oder einer jeweiligen von der Zählrate abhängigen, insbesondere proportionalen, Messgröße, insbesondere eines Werts der Messgröße, nur bzw. ausschließlich basierend auf jeweils erfassten Gammastrahlen, deren jeweilige Gamma-Energie, insbesondere deren jeweiliger Gamma-Energie-Wert, gleich oder größer als ein Energieschwellenwert ist, insbesondere und nicht kleiner, wobei der Energieschwellenwert minimal 0,5-mal eines Compton-Energiewerts einer Compton-Lücke der, insbesondere erfassten, Gammastrahlen ist.

Durch das erfindungsgemäße Verfahren kann erreicht werden, dass lediglich Gammastrahlen in die Auswertung eingehen, welche beim Auftreffen auf den Detektor noch eine hohe Energie haben, wobei somit davon ausgegangen werden kann, dass diese Gammastrahlen nicht oder nur sehr wenig gestreut haben. Erkannte Effekte des Übersprechens, bei welchen Gammastrahlen aus Strahlungsquellen emittiert werden bzw. austreten und vor Auftreffen auf einen Detektor so oft streuen, dass sie keinen vernünftigen Informationsgehalt mehr haben und unterschiedliche Schichten durchdrungen bzw. durchquert haben, können auf diese Weise wirkungsvoll vermieden werden. Bei Gammastrahlen mit hoher Energie, welche erfindungsgemäß ausgewertet werden, kann demgegenüber davon ausgegangen werden, dass sie nicht oder zumindest nur wenig gestreut haben und deshalb typischerweise nur eine Schicht durchdrungen bzw. durchquert haben, welche sie auch tatsächlich messen sollen. Dementsprechend genauer ist die Aussage über die jeweilige Schicht.

Eine Compton-Lücke ist in einem Spektrum einer jeweiligen Häufigkeit, insbesondere Zählrate, über einer jeweiligen Energie von Gammastrahlen nach Durchdringen bzw. Durchgehen durch einen Stoff typischerweise eine Lücke unmittelbar unterhalb eines Peaks, welcher die maximale, d.h. einer Emissions- bzw. Austrittsenergie aus der Strahlungsquelle entsprechende, Energie angibt. Erst unterhalb der Compton-Lücke, also bei niedrigeren Energien als der maximale Peak abzüglich der Compton-Lücke, treten wieder signifikante Häufigkeiten, insbesondere Zählraten, im Spektrum auf. Innerhalb der Compton-Lücke liegt der Compton-Energiewert, welcher typischerweise als Energiewert bei einer minimalen Häufigkeit, insbesondere Zählrate, innerhalb der Compton-Lücke oder auch als Minimum einer angefitteten, beispielsweise U-förmigen Funktion innerhalb der Compton-Lücke definiert werden kann. Dadurch wird insbesondere eine Abgrenzung zwischen Gammastrahlen, welche nicht gestreut haben und somit eine Energie oberhalb des Compton-Energiewerts haben, und Gammastrahlen, welche gestreut haben und somit eine Energie unterhalb des Compton-Energiewerts haben, erreicht.

Das Verfahren weist den folgenden Schritt auf: jeweils Emittieren von jeweiligen Gammastrahlen mit einer diskreten Isotop-Gamma-Energie, insbesondere einem diskreten Isotop-Gamma-Energie-Wert, in mindestens einen der Stoffe mittels einer Mehrzahl von Strahlungsquellen, wobei der Compton-Energiewert kleiner als die Isotop-Gamma-Energie ist. Entsprechend emittierte Gammastrahlen können für die hierin beschriebene Auswertung und allgemein für Messzwecke verwendet werden.

Gemäß einer Ausführung kann das Verfahren den folgenden Schritt aufweisen: c) insbesondere automatisches, Bestimmen des Dichteprofils, insbesondere von Werten des Dichteprofils, basierend auf den jeweils erzeugten Zählraten oder den jeweils erzeugten Messgrößen.

Gemäß einer Ausführung kann im Schritt a) jeweils das Erfassen Folgendes aufweisen: jeweils Erzeugen von jeweiligen Detektorsignalimpulsen mittels der Detektoren, wobei jeweilige Formen, insbesondere Amplituden und/oder Breiten und/oder Produkte der Amplituden und Breiten, der jeweils erzeugten Detektorsignalimpulse von den jeweiligen Gamma-Energien der jeweils, insbesondere erfassten, Gammastrahlen abhängig sind, insbesondere zu diesen proportional. Der Schritt b) kann Folgendes aufweisen: Jeweils Erzeugen der jeweiligen Zählrate oder der jeweilige Messgröße nur bzw. ausschließlich basierend auf jeweils erzeugten Detektorsignalimpulsen, deren jeweilige Formen gleich oder größer als ein Formenschwellenwert sind, wobei der Formenschwellenwert von dem Energieschwellenwert abhängig ist.

Unter dem Formenschwellenwert kann insbesondere ein abstrakter Schwellenwert verstanden werden, welcher auf einer Skala definiert wird, welche anzeigend für eine Form ist. Durch die Verwendung von Formen und Vergleich mit dem Formenschwellenwert können insbesondere Gammastrahlen besser erkannt werden, wobei beispielsweise am Detektor registrierte Ereignisse mit unüblicher Form aussortiert werden können. Dadurch kann beispielsweise die Messung auf Ereignisse beschränkt werden, welche auf Gammastrahlen basieren, die sich in gewünschter Weise von einer Strahlungsquelle durch eine Schicht zu einem Detektor durchdrungen bzw. fortbewegt haben. Zusätzlich oder alternativ kann der Formschwellenwert ein Amplitudenschwellenwert und/oder ein Breitenschwellenwert und/oder ein Produktschwellenwert sein.

Gemäß einer Ausführung können die Detektoren ein Detektor-Rauschen mit einem Rausch-Energiewert aufweisen, wobei der Energieschwellenwert minimal 2-mal der Rausch-Energiewert ist. Dadurch kann erreicht werden, dass Detektor-Rauschen automatisiert ausgefiltert wird, wobei vermeintliche Ereignisse, welche im Rauschen liegen, aufgrund ihrer zu geringen Energie ausgefiltert werden. Zusätzlich kann somit der Energieschwellenwert deutlich oberhalb eines, insbesondere bekannten, Energieschwellenwerts zum Ausfiltern von Detektor-Rauschen sein, welcher ein Eingehen häufig gestreuter Gammastrahlen in die Auswertung zulässt.

Zusätzlich oder alternativ kann der Energieschwellenwert minimal gleich dem Compton-Energiewert sein. Er kann insbesondere gleich dem Compton-Energiewert sein. Dadurch kann in vorteilhafter Weise erreicht werden, dass nur Ereignisse mit einer Energie oberhalb der Compton-Lücke tatsächlich ausgewertet werden und somit die Auswertung auf Gammastrahlen beschränkt wird, welche ohne Streuungsereignisse durch den jeweils zu messenden Stoff durchgegangen sind.

Gemäß einer Ausführung können die Detektoren seitlich zu einer, insbesondere runden, Wand des Behälters außerhalb angeordnet sein. Zusätzlich oder alternativ können die Detektoren vertikal übereinander angeordnet sein. Dadurch kann eine gute Anpassung an die Wand des Behälters erreicht werden, wobei beispielsweise die Detektoren bei der runden Wand entlang eines gebogenen Pfads angeordnet sein können, welcher beispielsweise eine Halbkreisform haben kann.

Gemäß einer bevorzugten Ausführung können die Strahlungsquellen innerhalb des Behälters angeordnet sein. Dadurch treten die aus den Strahlungsquellen austretenden Gammastrahlen unmittelbar in den jeweiligen Stoff ein.

Gemäß einer bevorzugten Ausführung können die Detektoren jeweils auf eine zugeordnete Strahlungsquelle gerichtet sein. Dadurch ist eine unmittelbare Zuordnung zwischen Detektoren und Strahlungsquellen gegeben, wobei typischerweise erwünscht ist, dass aus einer bestimmten Strahlungsquelle austretende Gammastrahlen nur von einem Detektor detektiert werden, typischerweise nach Durchgang durch einen bestimmten Stoff. Alternativ können die Detektoren auf einen Punkt, insbesondere mittig, zwischen zwei zugeordneten Strahlungsquellen gerichtet sein, wodurch beispielsweise eine "staggered"-Ausführung erreicht werden kann.

Gemäß einer bevorzugten Ausführung können die Detektoren jeweils auf gleicher Höhe wie die zugeordnete Strahlungsquelle angeordnet sein. Bei einer angenommenen horizontalen Ausrichtung jeweiliger Schichten von Stoffen kann somit in bevorzugter Weise erreicht werden, dass Gammastrahlen, welche nicht gestreut haben, tatsächlich nur durch einen Stoff durchgegangen sind, bevor sie den Detektor erreichen. Alternativ können die Detektoren jeweils auf gleicher Höhe wie der Punkt zwischen den zwei zugeordneten Strahlungsquellen angeordnet sein.

Gemäß einer Ausführung können die Detektoren jeweils einen Kollimator aufweisen, wobei die Kollimatoren jeweils einen Einfallswinkel einengen, insbesondere jeweils auf die zugeordnete/n Strahlungsquelle/n. Zusätzlich oder alternativ können die Strahlungsquellen jeweils einen Kollimator aufweisen, wobei die Kollimatoren jeweils einen Ausfallswinkel einengen, insbesondere jeweils auf den/die zugeordneten Detektor/en. Dadurch kann eine noch bessere Zuordnung zwischen Detektor und Strahlungsquelle/n erreicht werden, wobei insbesondere ein Übersprechen, insbesondere ungestreuter Gammas, zwischen Detektoren und Strahlungsquellen noch weiter verhindert werden kann.

Gemäß einer Ausführung können die Detektoren jeweils einen Szintillator zum Erfassen von jeweiligen Gammastrahlen aufweisen, wobei die Szintillatoren, insbesondere jeweils, eine Dichte von minimal 3 Gramm pro Kubikzentimeter (g/cm³), insbesondere von minimal 5 g/cm³, und/oder von maximal 20 g/cm³, insbesondere von maximal 10 g/cm³, insbesondere von 7 g/cm³, aufweisen bzw. haben können. Insbesondere können die Szintillatoren teilweise oder vollständig aus Elementen mit Ordnungszahl (Z) gleich oder größer als 31, 39, 48, 53, 55 oder 57 bestehen. Derartige Szintillatoren, insbesondere derartige Elemente, haben sich als vorteilhaft für die hier relevanten Detektionszwecke erwiesen, insbesondere da in diesen die jeweilige Energie von Gammastrahlen mit einer höheren Wahrscheinlichkeit vollständig deponiert wird. Insbesondere kann Bismutgermanat (BiGeO) und/oder Lanthanbromid (LaBr) und/oder Cäsiumiodid (Csl) und/oder Lutetiumyttriumoxyorthosilikat (LuYSiO) und/oder Cadmiumwolframat (CdWo) und/oder Gadoliniumaluminiumgalliumoxid (GdAlGaO) verwendet werden. Sie können beispielsweise alleine oder auch kombiniert in einem jeweiligen Detektor verwendet werden.

Gemäß einer Ausführung können die Stoffe Gas, Schaum, Öl, Emulsion, Wasser und/oder Sand aufweisen bzw. können dies sein. Zusätzlich oder alternativ kann der Behälter ein Öl-Wasser-Separator sein. Weiter zusätzlich oder alternativ können die Stoffe Gas, Kohlenwasserstoff und/oder Säure aufweisen bzw. sein. Weiter zusätzlich oder alternativ kann der Behälter ein Kohlenwasserstoff-Säure-Separator sein. Für derartige Anwendungen hat sich das erfindungsgemäße Verfahren als besonders vorteilhaft erwiesen. Insbesondere kann Gas Luft und/oder Kohlenwasserstoffe bezeichnen. Zusätzlich oder alternativ kann Schaum ein Gas-Öl-Gemisch bezeichnen. Weiter zusätzlich oder alternativ kann Emulsion ein Öl-Wasser-Gemisch bezeichnen.

Die Erfindung betrifft des Weiteren eine Vorrichtung zum Messen von, insbesondere den, Zählraten oder von, insbesondere den, den Zählraten abhängigen, insbesondere proportionalen, Messgrößen für ein Bestimmen eines, insbesondere des, Dichteprofils von, insbesondere den, mindestens zwei, insbesondere mindestens drei, Stoffen mit verschiedenen Dichten angeordnet innerhalb eines, insbesondere des, Behälters mittels einer, insbesondere der, Mehrzahl von Detektoren. Insbesondere kann die Vorrichtung zum Ausführen eines erfindungsgemäßen Verfahrens ausgebildet sein, wobei auf alle hierin beschriebenen Ausführungen und Varianten zurückgegriffen werden kann.

Die Vorrichtung weist eine Mehrzahl von, insbesondere elektrischen, Detektoren auf, wobei die Detektoren jeweils zum Erfassen von jeweiligen Gammastrahlen, welche jeweils mindestens einen der Stoffe mindestens teilweise durchdrungen haben, ausgebildet bzw. konfiguriert sind. Die Vorrichtung weist eine Mehrzahl von, insbesondere elektrischen, Erzeugungseinrichtungen auf, wobei die Erzeugungseinrichtungen jeweils zum Erzeugen einer jeweiligen Zählrate oder einer jeweiligen von der Zählrate abhängigen, insbesondere proportionalen, Messgröße nur basierend auf jeweils erfassten Gammastrahlen ausgebildet bzw. konfiguriert sind, deren jeweilige Gamma-Energie gleich oder größer als ein Energieschwellenwert ist, wobei der Energieschwellenwert minimal 0,5-mal eines Compton-Energiewerts einer Compton-Lücke der, insbesondere erfassten, Gammastrahlen ist.

Die Vorrichtung weist eine Mehrzahl von Strahlungsquellen auf, wobei die Strahlungsquellen jeweils zum Emittieren von jeweiligen Gammastrahlen mit einer diskreten Isotop-Gamma-Energie in mindestens einen der Stoffe ausgebildet bzw. konfiguriert sind, wobei der Compton-Energiewert kleiner als die Isotop-Gamma-Energie ist.

Mittels einer solchen Vorrichtung kann insbesondere das erfindungsgemäße Verfahren durchgeführt werden, und es können die weiter oben bereits beschriebenen Vorteile erreicht werden. Bezüglich der Vorrichtung kann auf alle Varianten zurückgegriffen werden, welche hierin mit Bezug auf das erfindungsgemäße Verfahren beschrieben sind.

Gemäß einer Ausführung kann die Vorrichtung eine Bestimmungseinrichtung aufweisen, wobei die Bestimmungseinrichtung zum Bestimmen des Dichteprofils basierend auf den jeweils erzeugten Zählraten oder den jeweils erzeugten Messgrößen ausgebildet bzw. konfiguriert ist. Die Bestimmungseinrichtung kann auch dazu ausgebildet sein, das erfindungsgemäße Verfahren auszuführen. Dadurch kann eine vorteilhafte Auswertung erreicht werden, wie dies bereits weiter oben beschrieben wurde.

Gemäß einer Ausführung kann die Vorrichtung den Behälter aufweisen.

Statt von einem Stoff kann beispielsweise auch von einem Material gesprochen werden.

Das Dichteprofil kann insbesondere ein vertikales Dichteprofil sein, d.h. die Dichte kann sich entlang einer vertikalen Richtung ändern, insbesondere aufgrund einer Trennung über die Gravitation. Es können beispielsweise Gas-, Flüssigkeits- und/oder Festkörper-Gemische geschichtet werden.

Es sei erwähnt, dass zusätzlich zu den hierin bereits beschriebenen Auswertungen auch weitere Auswertungen wie beispielsweise solche mit Bezug auf eine Temperatur möglich sind. Dadurch kann die Messgenauigkeit weiter verbessert werden.

Der Energieschwellenwert kann beispielsweise für alle Detektoren denselben Wert haben, es können jedoch auch unterschiedliche Werte verwendet werden.

Die Detektoren können insbesondere baugleich sein, sie können jedoch auch unterschiedlich ausgebildet sein.

Es sei erwähnt, dass beispielsweise zwei, drei, vier oder fünf Detektoren verwendet werden können. Auch mehr Detektoren können verwendet werden.

Es sei erwähnt, dass auch in Strahlungsquellen verwendete Isotope nicht nur eine Energie haben können, sondern beispielsweise auch zwei diskrete Energien haben können. Insbesondere kann der Energieschwellenwert basierend auf der höheren diskreten Energie gewählt bzw. eingestellt sein. Zusätzlich oder alternativ können die zwei diskreten Energien derart nah bei einander sein, dass sie als eine diskrete Energie bezüglich des Energieschwellenwerts behandelt werden können, wie z.B. für Kobalt 60. Es können auch Mischisotope verwendet werden.

Die Detektoren können beispielsweise jeweils einen Photomultiplier, insbesondere einen Silizium-Photomultiplier, aufweisen.

In dem Szintillator, soweit vorhanden, können durch Gammastrahlen jeweils mehrere Lichtblitze ausgelöst werden, deren Anzahl von der Energie der jeweiligen Gammastrahlen abhängt. Diese sehr schwachen Lichtblitze können aus einer Fotokathode eines dahinter angebrachten Photomultipliers, soweit vorhanden, Elektronen freisetzen. Diese Elektronen können durch Stöße an Elektroden im Photomultiplier lawinenartig vervielfacht werden. An einer Anode kann dann ein gut messbarer Stromimpuls abgenommen werden, insbesondere dessen Amplitude von der Energie der jeweiligen Gammastrahlen abhängig sein kann. Bei besonders kompakten Szintillationszählern kann anstelle des Photomultipliers auch eine empfindliche Fotodiode eingesetzt werden. Beispielsweise kann ein optoelektronischer Sensor verwendet werden.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind. Dabei zeigen:
- Fig. 1: eine Längsschnittansicht einer erfindungsgemäßen Vorrichtung aufweisend einen Behälter,
- Fig. 2: eine Querschnittansicht der Vorrichtung der Fig. 1 und eines erfindungsgemäßen Verfahrens,
- Fig. 3: eine schematische Ansicht der Vorrichtung der Fig. 1 und des Verfahrens der Fig. 2,
- Fig. 4: eine weitere schematische Ansicht der Vorrichtung der Fig. 1,
- Fig. 5: ein Energiespektrum von Gammastrahlen, und
- Fig. 6: eine weitere Ausführung der Vorrichtung der Fig. 1.

### DETAILLIERTE BESCHREIBUNG DER AUSFÜHRUNGSBEISPIELE

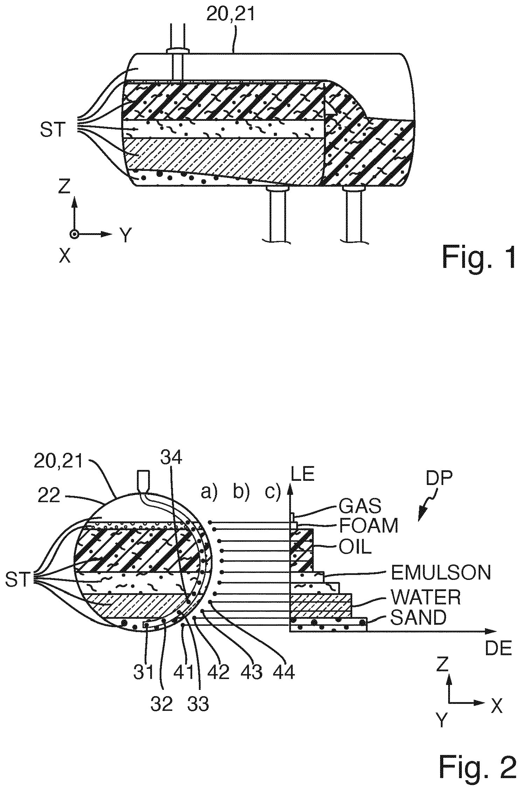

Fig. 1 bis 6 zeigen ein erfindungsgemäßes Verfahren und eine erfindungsgemäße Vorrichtung 10 zum Messen von Zählraten ZR1, ZR2, ZR3, ZR4 für ein Bestimmen eines Dichteprofils DP von mindestens zwei Stoffen ST mit verschiedenen Dichten DE angeordnet innerhalb eines Behälters 20 mittels einer Mehrzahl von Detektoren 41, 42, 43, 44.

Im Detail weist die Vorrichtung 10 den Behälter 20 auf, welcher vorliegend als Öl-Wasser-Separator 21 ausgebildet ist. Dabei liegen die Stoffe ST schichtartig übereinander. Der Behälter 20 dient grundsätzlich zum Trennen dieser Stoffe ST. In Fig. 1 ist auch ein Koordinatensystem eingezeichnet, welches x-Richtung, y-Richtung und z-Richtung definiert.

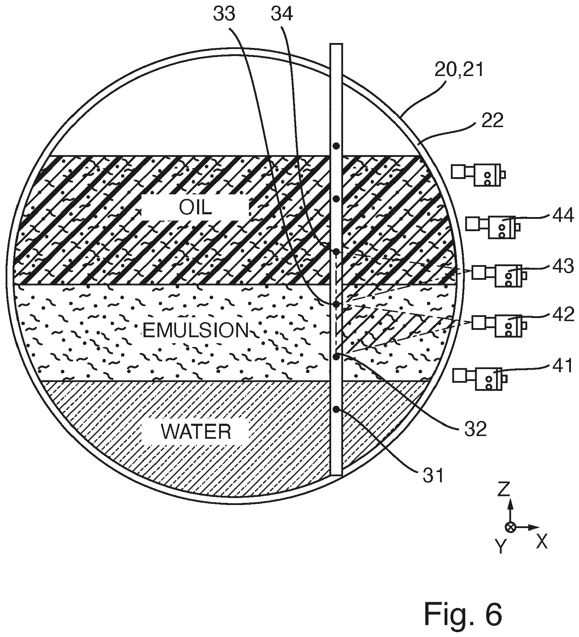

Fig. 2 und 6 zeigen den Behälter 20 im Querschnitt mit den darin befindlichen Stoffen ST und eine danebenliegende Auflistung der verschiedenen Stoffe ST. Es ist dabei zu erkennen, dass der Behälter 20 einen runden Querschnitt hat. Dieser wird von einer Wand 22 definiert.

Die Stoffe ST bzw. die erkennbaren Schichten in dem Behälter 20 sind vorliegend, von unten nach oben bzw. in der z-Richtung, Sand (SAND), Wasser (WATER), eine Emulsion (EMULSION), Öl (OIL), Schaum (FOAM) und Gas (GAS). Es sei verstanden, dass derartige Stoffe ST lediglich beispielhaft sind.

Innerhalb des Behälters 20 ist eine Mehrzahl von Strahlungsquellen 31, 32, 33, 34 der Vorrichtung 10 angeordnet, von welchen hier lediglich einige schematisch bezeichnet sind. Wie in Fig. 2 bis 4 gezeigt sind die Strahlungsquellen 31, 32, 33, 34 entlang eines kreisbogenförmigen Segments vertikal übereinander bzw. in der z-Richtung angeordnet und haben jeweils in radialer x-Richtung gesehen einen ähnlichen Abstand zur Wand 22. Rechtsseitig zu der, insbesondere runden, Wand 22 bzw. in x-Richtung außerhalb des Behälters 20 ist eine Mehrzahl von Detektoren 41, 42, 43, 44 der Vorrichtung 10 angeordnet, von welchen hier lediglich einige schematisch bezeichnet sind. Auch diese sind entlang eines kreisbogenförmigen Segments vertikal übereinander bzw. in der z-Richtung angeordnet. Die Detektoren 41, 42, 43, 44 können somit in einem Schritt a) des Verfahrens jeweils jeweilige Gammastrahlen GR erfassen, welche jeweils von den Strahlungsquellen 31, 32, 33, 34 emittiert worden sind und den jeweiligen Stoff ST mindestens teilweise durchdrungen haben.

Rechts in Fig. 2 ist das Dichteprofil DP der Stoffe ST dargestellt. Dabei ist über der Höhe LE die jeweilige Dichte DE eines der Stoffe ST angetragen. Es ist zu erkennen, dass die Dichte DE grundsätzlich von oben nach unten bzw. entgegen der z-Richtung zunimmt und die Stoffe ST entsprechend separiert sind.

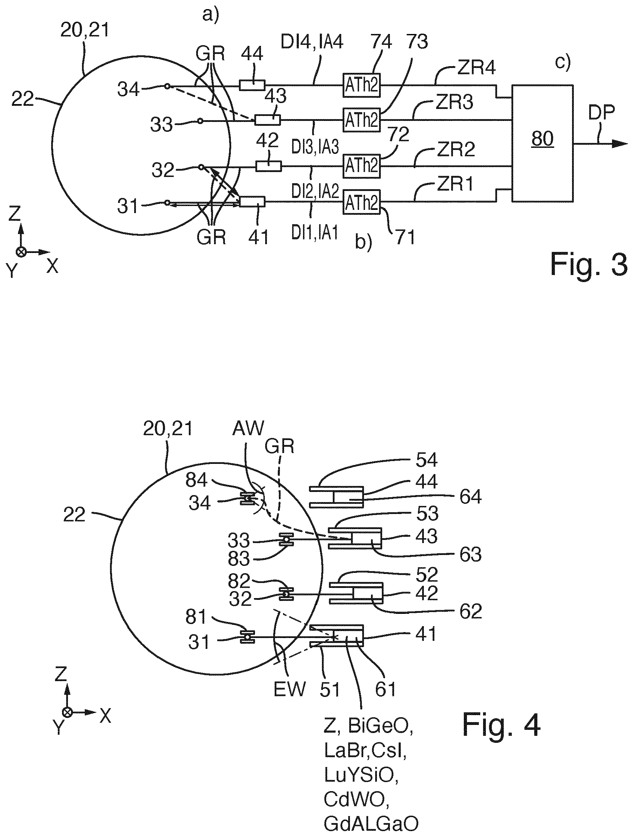

Fig. 3 zeigt im Detail Mechanismen zur Auswertung und eine zugehörige schematische Signalverarbeitung. Dabei sind nur noch die bereits erwähnten vier Detektoren 41, 42, 43, 44 und die zugeordneten Strahlungsquellen 31, 32, 33, 34 stellvertretend für die Gesamtzahl an Detektoren und Strahlungsquellen dargestellt.

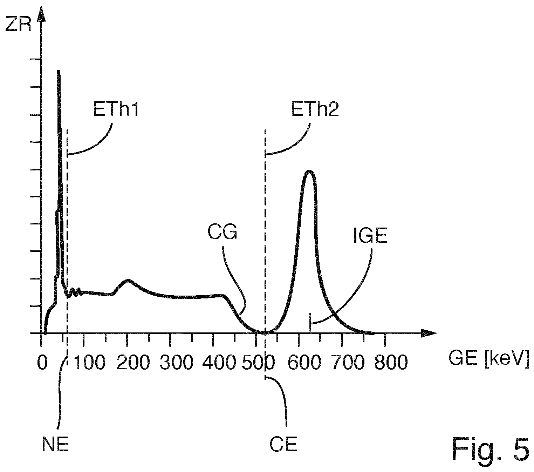

Mittels einer Mehrzahl von Erzeugungseinrichtungen 71, 72, 73, 74 der Vorrichtung 10 werden in einem Schritt b) des Verfahrens jeweilige Zählraten ZR1, ZR2, ZR3, ZR4 nur basierend auf jeweils erfassten Gammastrahlen GR erzeugt bzw. generiert, deren jeweilige Gamma-Energie GE gleich oder größer als ein Energieschwellenwert ETh2 sind, wobei der Energieschwellenwert ETh2 minimal 0,5-mal eines Compton-Energiewerts CE einer Compton-Lücke CG der, insbesondere erfassten, Gammastrahlen GR ist, wie in Fig. 5 gezeigt.

Im Detail erzeugen die Detektoren 41, 42, 43, 44 in dem Schritt a) jeweils jeweilige Detektorsignalimpulse DI1, DI2, DI3, DI4, wobei jeweilige Formen, insbesondere Amplituden IA1, IA2, IA3, IA4, der jeweils erzeugten Detektorsignalimpulse DI1, DI2, DI3, DI4 von den jeweiligen Gamma-Energien GE der jeweils, insbesondere erfassten, Gammastrahlen GR abhängig sind. Dabei werden insbesondere jeweilige Formen, insbesondere Amplituden IA1, IA2, IA3, IA4, der jeweils erzeugten Detektorsignalimpulse DI1, DI2, DI3, DI4 ausgewertet. Die jeweiligen Formen, insbesondere die jeweiligen Amplituden IA1, IA2, IA3, IA4, werden jeweils mit einem jeweiligen Formenschwellenwert ATh2 verglichen, wobei der Formenschwellenwert ATh2 von dem Energieschwellenwert ETh2 abhängig ist. Nur basierend auf jeweils erzeugten Detektorsignalimpulsen DI1, DI2, DI3, DI4 bzw. Ereignissen, deren jeweiligen Formen, insbesondere deren jeweilige Amplituden IA1, IA2, IA3, IA4, gleich oder größer als der Formenschwellenwert ATh2 sind, werden die jeweiligen Zählraten ZR1, ZR2, ZR3, ZR4 erzeugt bzw. ausgewertet.

Vorliegend ist der Energieschwellenwert ETh2 gleich dem Compton-Energiewert CE der Compton-Lücke CG der verwendeten Gammastrahlen GR, also den von den Strahlungsquellen 31, 32, 33, 34 emittierten Gammastrahlen GR.

Basierend auf den Zählraten ZR1, ZR2, ZR3, ZR4 wird anschließend in einem Schritt c) des Verfahrens mittels einer Bestimmungseinrichtung 80 der Vorrichtung 10 das Dichteprofil DP bestimmt.

Dadurch kann eine jeweilige Dichte DE zwischen jeweiliger Strahlungsquelle 31, 32, 33, 34 und jeweiligem Detektor 41, 42, 43, 44 in vorteilhafter Weise ermittelt werden, da nahezu ausschließlich ungestreute Gammastrahlen GR ausgewertet werden. Übersprecheffekte werden dadurch vermieden.

Der Effekt des Übersprechens ist in Fig. 3 dargestellt. Dabei sind jeweilige Gammastrahlen GR eingezeichnet, welche unmittelbar horizontal von der jeweiligen Strahlungsquelle 31, 32, 33, 34 zum jeweiligen Detektor 41, 42, 43, 44 laufen, und zwar mit einer jeweiligen durchgezogenen Linie. Es sind jedoch auch gestrichelt eingezeichnete Strahlungsverläufe von Gammastrahlen GR eingezeichnet, welche ein Übersprechen darstellen. Derartige Gammastrahlen GR sind für die Auswertung typischerweise unerwünscht, da sie gestreut haben und typischerweise nicht nur durch einen Stoff ST durchdrungen haben. Sie liefern somit keinen Beitrag zu einer zuverlässigen Dichtebestimmung. Dieser Effekt ist besonders stark bei runden Geometrien, tritt jedoch auch bei anderen Geometrien auf.

In Fig. 4 ist ein Teil der Vorrichtung 10 dargestellt, wobei die spezifischen Mechanismen zur Auswertung nicht mehr dargestellt sind. Die Detektoren 41, 42, 43, 44 weisen dabei jeweilige Kollimatoren 51, 52, 53, 54 auf, welche dafür sorgen, dass ein Einfallswinkel bzw. Erfassungswinkel EW des jeweiligen Detektors 41, 42, 43, 44 eingeengt wird, insbesondere auf die jeweils zugeordnete Strahlungsquelle 31, 32, 33, 34. Zusätzlich weisen die Strahlungsquellen 31, 32, 33, 34 jeweilige Kollimatoren 81, 82, 83, 84 auf, welche dafür sorgen, dass ein Ausfallswinkel AW der jeweiligen Strahlungsquelle 31, 32, 33, 35 eingeengt wird, insbesondere auf den jeweils zugeordneten Detektor 41, 42, 43, 44. Dadurch können Übersprecheffekte zwar verringert, jedoch nicht vollständig vermieden werden, wie beispielsweise durch einen gestrichelt eingezeichneten möglichen Verlauf eines mehrfach gestreuten Gammastrahls bzw. Gammaquants GR leicht zu erkennen ist.

Der jeweilige Einfallswinkel ist dabei mit dem Bezugszeichen EW dargestellt, und zwar beispielhaft bei dem untersten Detektor 41. Der jeweilige Ausfallswinkel ist dabei mit dem Bezugszeichen AW dargestellt, und zwar beispielhaft bei der obersten Strahlungsquelle 34.

Die Detektoren 41, 42, 43, 44 weisen jeweils einen Szintillator 61, 62, 63, 64 zum Erfassen von jeweiligen Gammastrahlen GR auf, insbesondere welche bei Auftreffen von Gammastrahlen GR jeweilige Lichtblitze abgeben. Diese Lichtblitze werden dann typischerweise durch Photomultiplier verstärkt und entsprechend ausgewertet. Die Szintillatoren 61, 62, 63, 64 können eine Dichte von minimal 3 g/cm³ und/oder von maximal 20 g/cm³ aufweisen. Wie gezeigt können die Szintillatoren 61, 62, 63, 64 teilweise oder vollständig beispielsweise aus einem Element mit hoher Ordnungszahl Z, insbesondere gleich oder größer als 31 bestehen, wobei beispielsweise BiGeO, LaBr, Csl, LuYSiO, CdWO oder GdAlGaO verwendet werden kann.

Fig. 5 zeigt ein typisches Energiespektrum, welches von einem der Detektoren 41, 42, 43, 44 detektiert wird. Auf der horizontalen Achse ist dabei die jeweilige Energie GE der Gammastrahlen GR in der Einheit keV angetragen, an der vertikalen Achse ist die jeweilige Zählrate ZR pro Energie bzw. Energiekanal angetragen.

Bei kleinen Energien ist zunächst ein elektronisches Rauschen zu erkennen, welches unterhalb eines anderen Energieschwellenwerts ETh1 liegt. Das Rauschen endet beim Rausch-Energiewert NE.

In dem gezeigten Ausführungsbeispiel bei Verwendung von Cäsium 137 (Cs 137) für die Strahlungsquellen 31, 32, 33, 34 ist zwischen etwa 450 keV und 600 keV die Compton-Lücke CG angeordnet, in welcher die Zählrate ZR nahezu null wird. Mittig innerhalb dieser Compton-Lücke CG befindet sich der Compton-Energiewert CE, welchem vorliegend der Energieschwellenwert ETh2 gleich ist. Darüber befindet sich ein Peak mit einem ausgeprägten Maximum bei einer diskreten Isotop-Gamma-Energie IGE der emittierten Gammastrahlen GR aus den Strahlungsquellen 31, 32, 33, 34. In anderen Worten: der Compton-Energiewert CE ist kleiner als die Isotop-Gamma-Energie IGE. In alternativen Ausführungsbeispielen können die Detektoren jeweils einen Szintillator, insbesondere einen organischen Szintillator, aufweisen, mittels welcher ein detektiertes Energiespektrum einen weniger ausgeprägten Peak oder keinen Peak bei einer diskreten Isotop-Gamma-Energie aufweist bzw. hat. Anders formuliert: eine signifikante Häufigkeit, insbesondere Zählrate, kann mit der Compton-Lücke enden.

Würde man in dem gezeigten Ausführungsbeispiel den anderen Energieschwellenwert ETh1 verwenden, würde dieser alle Gammastrahlen GR detektieren, welche auf oder oberhalb der Rauschschwelle liegen. Dies kann zu den bereits weiter oben beschriebenen unerwünschten Effekten führen, da insbesondere auch Gammastrahlen GR detektiert werden, welche mehrfach gestreut haben und durch Schichten durchgegangen sind, welche gar nicht gemessen werden sollen. Wird dahingegen der Energieschwellenwert ETh2 verwendet, insbesondere welcher minimal 2-mal der Rausch-Energiewert NE ist, so werden nur noch die ungestreuten Gammastrahlen GR ausgewertet, welche beispielsweise bei der mit Bezug auf die Fig. 2 bis 4 beschriebenen Anordnung typischerweise horizontal durch nur einen Stoff ST durchgegangen sind. Diese besitzen typischerweise die volle Energie der Strahlungsquellen 31, 32, 33, 34, welche beispielsweise bei Verwendung von Cäsium 137 einen Wert von 662 keV hat. Mit dieser Selektion werden, beispielsweise unterstützt durch die Verwendung von den Kollimatoren 51, 52, 53, 54, 81, 82, 83, 84, auch Gammastrahlen bzw. Gammaquanten GR, die nach mindestens einer Streuung an einem der Detektoren 41, 42, 43, 44 angekommen sind, ausgefiltert, so dass auf jeden Fall eine jeweils gute Zuordnung zwischen den Strahlungsquellen 31, 32, 33, 34 und den Detektoren 41, 42, 43, 44 gegeben ist. Dies ermöglicht ein deutlich verbessertes Messergebnis, was wiederum ermöglicht, dass eine Anlage deutlich näher an der Designgrenze betrieben werden kann, ohne beispielsweise bei dem Öl-Wasser-Separator 21 das Risiko zu haben, Rohöl in das Abwasser abzuleiten.

Die Eigenschaften der Compton-Streuung sind grundsätzlich bekannt, so dass der Energieverlust des Gammastrahlen GR direkt auf einen Streuwinkel zurückschließen lässt. Dies erlaubt aber umgekehrt auch Streuungen, die nur unter einem kleinen Winkel stattgefunden haben und damit die jeweilige Zuordnung von Strahlungsquellen 31, 32, 33, 34 und Detektoren 41, 42, 43, 44 noch nicht gefährdet haben, weiter zuzulassen. Dies ist möglich, indem man die Messschwelle nicht direkt unterhalb des Peaks mit maximaler Energie legt, sondern weiter darunter. Je weiter man die Schwelle nach unten versetzt, desto mehr gestreute Gammastrahlen GR werden detektiert. Damit hat man einen Stellhebel zwischen erlaubtem Übersprechen (= Messgenauigkeit) und Zählrateneffizienz. Man kann die erfindungsgemäße Lösung daher je nach Applikation optimieren und anpassen.

Da die Gammastrahlen bzw. Gammaquanten GR in den jeweiligen Szintillatoren 61, 62. 63, 64 sowohl über den Fotoeffekt als auch über die Compton-Streuung detektiert werden, kann es sein, dass auch Gammastrahlen GR mit voller Energie IGE nur einen Bruchteil der Energie IGE im jeweiligen Szintillator 61, 62. 63, 64 deponieren könnten. Dadurch würden sie fälschlicherweise als Gammastrahlen GR mit zu niedriger Energie detektiert. Diesem Effekt kann man entgegenwirken, indem man das Detektormaterial mit hoher Dichte, insbesondere mit Elementen mit hoher Ordnungszahl Z, verwendet. Dann überwiegt der Fotoeffekt gegenüber der Compton-Streuung, womit die volle Energie, insbesondere IGE, detektiert wird. Die geringe Zählrateneffizienz kann weiterhin durch eine Erhöhung der Quellenaktivität kompensiert werden.

Oft dürfte aber auch eine Erhöhung der Mittelungszeit ausreichen, da die Prozesse in Separatoren ohnehin nicht schnell ablaufen.

Fig. 6 zeigt noch eine alternative Konfiguration des Behälters 20 mit den Strahlungsquellen 31, 32, 33, 34 und Detektoren 41, 42, 43, 44. Im Unterschied zu der Ausführung der Fig. 2 bis 4 sind die Detektoren 41, 42, 43, 44 dabei nicht mehr jeweils auf eine zugeordnete Strahlungsquelle 31, 32, 33, 34 gerichtet, insbesondere auf gleicher Höhe in der z-Richtung wie die zugehörige Strahlungsquelle 31, 32, 33, 34 angeordnet, sondern sind vielmehr auf einen Punkt, insbesondere mittig, zwischen zwei zugeordneten Strahlungsquellen 31, 32, 33, 34 gerichtet, insbesondere auf gleicher Höhe in der Richtung z wie der Punkt zwischen den zwei zugeordneten Strahlungsquellen 31, 32, 33, 34 bzw. in der z-Richtung gesehen versetzt dazu angeordnet.

## Patentansprüche

1. Verfahren zum Messen von Zählraten (ZR1, ZR2, ZR3, ZR4) oder von den Zählraten abhängigen Messgrößen für ein Bestimmen eines Dichteprofils (DP) von mindestens zwei Stoffen (ST) mit verschiedenen Dichten (DE) angeordnet innerhalb eines Behälters (20) mittels einer Mehrzahl von Detektoren (41, 42, 43, 44), wobei das Verfahren die Schritte aufweist:
a) jeweils Erfassen von jeweiligen Gammastrahlen (GR), welche jeweils mindestens einen der Stoffe (ST) mindestens teilweise durchdrungen haben, mittels der Detektoren (41, 42, 43, 44), und
b) jeweils Erzeugen einer jeweiligen Zählrate (ZR1, ZR2, ZR3, ZR4) oder einer jeweiligen von der Zählrate abhängigen Messgröße nur basierend auf jeweils erfassten Gammastrahlen (GR), deren jeweilige Gamma-Energie (GE) gleich oder größer als ein Energieschwellenwert (ETh2) sind, wobei der Energieschwellenwert (ETh2) minimal 0,5-mal eines Compton-Energiewerts (CE) einer Compton-Lücke (CG) der Gammastrahlen (GR) ist,
- wobei das Verfahren den Schritt aufweist: jeweils Emittieren von jeweiligen Gammastrahlen (GR) mit einer diskreten Isotop-Gamma-Energie (IGE) in mindestens einen der Stoffe (ST) mittels einer Mehrzahl von Strahlungsquellen (31, 32, 33, 34), wobei der Compton-Energiewert (CE) kleiner als die Isotop-Gamma-Energie (IGE) ist.

2. Verfahren nach Anspruch 1, wobei das Verfahren den Schritt aufweist:
c) Bestimmen des Dichteprofils (DP) basierend auf den jeweils erzeugten Zählraten (ZR1, ZR2, ZR3, ZR4) oder den jeweils erzeugten Messgrößen.

3. Verfahren nach einem der vorhergehenden Ansprüche,
- wobei im Schritt a) jeweils das Erfassen aufweist: jeweils Erzeugen von jeweiligen Detektorsignalimpulsen (DI1, DI2, DI3, D14) mittels der Detektoren (41, 42, 43, 44), wobei jeweilige Formen, insbesondere Amplituden (IA1, IA2, IA3, IA4) und/oder Breiten und/oder Produkte der Amplituden und Breiten, der jeweils erzeugten Detektorsignalimpulse (DI1, DI2, DI3, DI4) von den jeweiligen Gamma-Energien (GE) der jeweils Gammastrahlen (GR) abhängig sind, und
- wobei der Schritt b) aufweist: jeweils Erzeugen der jeweiligen Zählrate (ZR1, ZR2, ZR3, ZR4) oder der jeweiligen Messgröße nur basierend auf jeweils erzeugten Detektorsignalimpulsen (DI1, DI2, DI3, DI4), deren jeweilige Formen gleich oder größer als ein Formenschwellenwert (ATh2) sind, wobei der Formenschwellenwert (ATh2) von dem Energieschwellenwert (ETh2) abhängig ist.

4. Verfahren nach einem der vorhergehenden Ansprüche,
- wobei die Detektoren (41, 42, 43 44) ein Detektor-Rauschen mit einem Rausch-Energiewert (NE) aufweisen, wobei der Energieschwellenwert (ETh2) minimal 2-mal der Rausch-Energiewert (NE) ist, und/oder
- wobei der Energieschwellenwert (ETh2) minimal gleich dem Compton-Energiewert (CE) ist.

5. Verfahren nach einem der vorhergehenden Ansprüche,
- wobei die Detektoren (41, 42, 43, 44) seitlich zu einer, insbesondere runden, Wand (22) des Behälters (20) außerhalb angeordnet ist, und/oder
- wobei die Detektoren (41, 42, 43, 44) vertikal übereinander angeordnet sind.

6. Verfahren nach einem der vorhergehenden Ansprüche, insbesondere Anspruch 5,
- wobei die Strahlungsquellen (31, 32, 33, 34) innerhalb des Behälters (20) angeordnet sind.

7. Verfahren nach einem der vorhergehenden Ansprüche, insbesondere Anspruch 6,
- wobei die Detektoren (41, 42, 43, 44) jeweils auf eine zugeordnete Strahlungsquelle (31, 32, 33, 34) oder auf einen Punkt, insbesondere mittig, zwischen zwei zugeordneten Strahlungsquellen (31, 32, 33, 34) gerichtet sind.

8. Verfahren nach Anspruch 7, insbesondere und nach Anspruch 5,
- wobei die Detektoren (41, 42, 43, 44) jeweils auf gleicher Höhe wie die zugeordnete Strahlungsquelle (31, 32, 33, 34) oder auf gleicher Höhe wie der Punkt zwischen den zwei zugeordneten Strahlungsquellen (31, 32, 33, 34) angeordnet sind.

9. Verfahren nach einem der vorhergehenden Ansprüche, insbesondere nach einem der Ansprüche 6 bis 8,
- wobei die Detektoren (41, 42, 43, 44) jeweils einen Kollimator (51, 52, 53, 54) aufweisen, wobei die Kollimatoren (51, 52, 53, 54) jeweils einen Einfallswinkel (EW) einengen, insbesondere jeweils auf die zugeordnete/n Strahlungsquelle/n (31, 32, 33, 34), und/oder
- wobei die Strahlungsquellen (31, 32, 33, 34) jeweils einen Kollimator (81, 82, 83, 84) aufweisen, wobei die Kollimatoren (81, 82, 83, 84) jeweils einen Ausfallswinkel (AW) einengen, insbesondere jeweils auf den/die zugeordneten Detektor/en (41, 42, 43, 44).

10. Verfahren nach einem der vorhergehenden Ansprüche,
- wobei die Detektoren (41, 42, 43, 44) jeweils einen Szintillator (61, 62, 63, 64) zum Erfassen von jeweiligen Gammastrahlen (GR) aufweisen, wobei die Szintillatoren (61, 62, 63, 64) eine Dichte von minimal 3 g/cm³, insbesondere von minimal 5 g/cm³, und/oder von maximal 20 g/cm³, insbesondere von maximal 10 g/cm³, insbesondere von 7 g/cm³, aufweisen.

11. Verfahren nach einem der vorhergehenden Ansprüche,
- wobei die Stoffe (ST) Gas (GAS), Schaum (FOAM), Öl (OIL), Emulsion (EMULSION) Wasser (WATER) und/oder Sand (SAND) aufweisen, insbesondere sind, und/oder wobei der Behälter (20) ein Öl-Wasser-Separator (21) ist, und/oder
- wobei die Stoffe Gas, Kohlenwasserstoff und/oder Säure aufweisen, und/oder wobei der Behälter ein Kohlenwasserstoff-Säure-Separator ist.

12. Vorrichtung (10) zum Messen von Zählraten (ZR1, ZR2, ZR3, ZR4) oder von den Zählraten abhängigen Messgrößen für ein Bestimmen eines Dichteprofils (DP) von mindestens zwei Stoffen (ST) mit verschiedenen Dichten (DE) angeordnet innerhalb eines Behälters (20) mittels einer Mehrzahl von Detektoren (41, 42, 43, 44), insbesondere zum Ausführen eines Verfahren nach einem der vorhergehenden Ansprüche, wobei die Vorrichtung (10) aufweist:
- die Mehrzahl von Detektoren (41, 42, 43, 44), wobei die Detektoren (41, 42, 43, 44) jeweils zum Erfassen von jeweiligen Gammastrahlen (GR), welche jeweils mindestens einen der Stoffe (ST) mindestens teilweise durchdrungen haben, ausgebildet sind, und
- eine Mehrzahl von Erzeugungseinrichtungen (71, 72, 73, 74), wobei die Erzeugungseinrichtungen (71, 72, 73, 74) jeweils zum Erzeugen einer jeweiligen Zählrate (ZR1, ZR2, ZR3, ZR4) oder einer jeweiligen von der Zählrate abhängigen Messgröße nur basierend auf jeweils erfassten Gammastrahlen (GR) ausgebildet sind, deren jeweilige Gamma-Energie (GE) gleich oder größer als ein Energieschwellenwert (ETh2) sind, wobei der Energieschwellenwert (ETh2) minimal 0,5-mal eines Compton-Energiewerts (CE) einer Compton-Lücke (CG) der Gammastrahlen (GR) ist,
- wobei die Vorrichtung (10) aufweist: eine Mehrzahl von Strahlungsquellen (31, 32, 33, 34), wobei die Strahlungsquellen (31, 32, 33, 34) jeweils zum Emittieren von jeweiligen Gammastrahlen (GR) mit einer diskreten Isotop-Gamma-Energie (IGE) in mindestens einen der Stoffe (ST) ausgebildet sind, wobei der Compton-Energiewert (CE) kleiner als die Isotop-Gamma-Energie (IGE) ist.

13. Vorrichtung (10) nach Anspruch 12, wobei die Vorrichtung (10) aufweist:
- eine Bestimmungseinrichtung (80), wobei die Bestimmungseinrichtung (80) zum Bestimmen des Dichteprofils (DP) basierend auf den jeweils erzeugten Zählraten (ZR1, ZR2, ZR3, ZR4) oder den jeweils erzeugten Messgrößen ausgebildet ist.

14. Vorrichtung (10) nach Anspruch 12 oder 13, wobei die Vorrichtung (10) aufweist:
- den Behälter (20).

## Claims

1. Method for measuring counting rates (ZR1, ZR2, ZR3, ZR4) or measured variables dependent on the counting rates for determining a density profile (DP) of at least two substances (ST) with different densities (DE) arranged within a container (20) by means of a plurality of detectors (41, 42, 43, 44), the method comprising the steps of:
a) in each case recording respective gamma rays (GR) which have in each case penetrated at least partially through at least one of the substances (ST), by means of the detectors (41, 42, 43, 44), and
b) in each case generating a respective counting rate (ZR1, ZR2, ZR3, ZR4) or a respective measured variable dependent on the counting rate only on the basis of respectively recorded gamma rays (GR) of which the respective gamma energy (GE) is greater than or equal to an energy threshold value (ETh2), the energy threshold value (ETh2) being a minimum of 0.5 times a Compton energy value (CE) of a Compton gap (CG) of the gamma rays (GR),
- the method comprising the step of: in each case emitting respective gamma rays (GR) with a discrete isotope gamma energy (IGE) into at least one of the substances (ST) by means of a plurality of radiation sources (31, 32, 33, 34), the Compton energy value (CE) being less than the isotope gamma energy (IGE).

2. Method according to Claim 1, the method comprising the step of:
c) determining the density profile (DP) on the basis of the respectively generated counting rates (ZR1, ZR2, ZR3, ZR4) or the respectively generated measured variables.

3. Method according to either of the preceding claims,
- in step a), the recording in each case comprising: in each case generating respective detector signal pulses (DI1, DI2, DI3, DI4) by means of the detectors (41, 42, 43, 44), with respective forms, in particular amplitudes (IA1, IA2, IA3, IA4) and/or widths and/or products of the amplitudes and widths, of the respectively generated detector signal pulses (DI1, DI2, DI3, DI4) being dependent on the respective gamma energy levels (GE) of the respective gamma rays (GR), and
- step b) comprising: in each case generating the respective counting rate (ZR1, ZR2, ZR3, ZR4) or the respective measured variable only on the basis of respectively generated detector signal pulses (DI1, DI2, DI3, DI4) of which the respective forms are equal to or greater than a form threshold value (ATh2), the form threshold value (ATh2) being dependent on the energy threshold value (ETh2).

4. Method according to one of the preceding claims,
- the detectors (41, 42, 43, 44) having a detector noise with a noise energy value (NE), the energy threshold value (ETh2) being a minimum of 2 times the noise energy value (NE), and/or
- the energy threshold value (ETh2) being a minimum of equal to the Compton energy value (CE).

5. Method according to one of the preceding claims,
- the detectors (41, 42, 43, 44) being arranged laterally outside a wall (22), in particular a round wall (22), of the container (20), and/or
- the detectors (41, 42, 43, 44) being arranged vertically one above the other.

6. Method according to one of the preceding claims, in particular Claim 5,
- the radiation sources (31, 32, 33, 34) being arranged within the container (20).

7. Method according to one of the preceding claims, in particular Claim 6,
- the detectors (41, 42, 43, 44) being directed in each case at an assigned radiation source (31, 32, 33, 34) or at a point between, in particular midway between, two assigned radiation sources (31, 32, 33, 34).

8. Method according to Claim 7, in particular according to Claim 5,
- the detectors (41, 42, 43, 44) being arranged in each case at the same height as the assigned radiation source (31, 32, 33, 34) or at the same height as the point between the two assigned radiation sources (31, 32, 33, 34) .

9. Method according to one of the preceding claims, in particular according to one of Claims 6 to 8,
- the detectors (41, 42, 43, 44) in each case comprising a collimator (51, 52, 53, 54), the collimators (51, 52, 53, 54) respectively narrowing an angle of incidence (EW), in particular in each case to the assigned radiation source(s) (31, 32, 33, 34), and/or
- the radiation sources (31, 32, 33, 34) in each case comprising a collimator (81, 82, 83, 84), the collimators (81, 82, 83, 84) respectively narrowing an angle of emergence (AW), in particular in each case to the assigned detector(s) (41, 42, 43, 44).

10. Method according to one of the preceding claims,
- the detectors (41, 42, 43, 44) in each case comprising a scintillator (61, 62, 63, 64) for recording respective gamma rays (GR), the scintillators (61, 62, 63, 64) comprising a density of a minimum of 3 g/cm³, in particular a minimum of 5 g/cm³, and/or a maximum of 20 g/cm³, in particular a maximum of 10 g/cm³, in particular of 7 g/cm³.

11. Method according to one of the preceding claims,
- the substances (ST) comprising, in particular being, gas (GAS), foam (FOAM), oil (OIL), emulsion (EMULSION), water (WATER) and/or sand (SAND), and/or the container (20) being an oil-water separator (21), and/or
- the substances comprising gas, hydrocarbon and/or acid, and/or the container being a hydrocarbon-acid separator.

12. Apparatus (10) for measuring counting rates (ZR1, ZR2, ZR3, ZR4) or measured variables dependent on the counting rates for determining a density profile (DP) of at least two substances (ST) with different densities (DE) arranged within a container (20) by means of a plurality of detectors (41, 42, 43, 44), in particular for performing a method according to one of the preceding claims, the apparatus (10) comprising:
- the plurality of detectors (41, 42, 43, 44), the detectors (41, 42, 43, 44) being designed in each case for recording respective gamma rays (GR) which have in each case penetrated at least partially through at least one of the substances (ST), and
- a plurality of generating devices (71, 72, 73, 74), the generating devices (71, 72, 73, 74) being designed in each case for generating a respective counting rate (ZR1, ZR2, ZR3, ZR4) or a respective measured variable dependent on the counting rate only on the basis of respectively recorded gamma rays (GR) of which the respective gamma energy (GE) is greater than or equal to an energy threshold value (ETh2), the energy threshold value (ETh2) being a minimum of 0.5 times a Compton energy value (CE) of a Compton gap (CG) of the gamma rays (GR),
- the apparatus (10) comprising: a plurality of radiation sources (31, 32, 33, 34), the radiation sources (31, 32, 33, 34) being designed in each case for emitting respective gamma rays (GR) with a discrete isotope gamma energy (IGE) into at least one of the substances (ST), the Compton energy value (CE) being less than the isotope gamma energy (IGE).

13. Apparatus (10) according to Claim 12, the apparatus (10) comprising:
- a determining device (80), the determining device (80) being designed for determining the density profile (DP) on the basis of the respectively generated counting rates (ZR1, ZR2, ZR3, ZR4) or the respectively generated measured variables.

14. Apparatus (10) according to Claim 12 or 13, the apparatus (10) comprising:
- the container (20).

## Revendications

1. Procédé de mesure de taux de comptage (ZR1, ZR2, ZR3, ZR4) ou de grandeurs mesurées dépendant des taux de comptage permettant une détermination d'un profil de densité (DP) d'au moins deux substances (ST) de densités différentes (DE) disposées à l'intérieur d'un récipient (20) au moyen d'une pluralité de détecteurs (41, 42, 43, 44), le procédé comportant les étapes consistant à :
a) détecter respectivement, au moyen des détecteurs (41, 42, 43, 44), des rayons gamma respectifs (GR) qui ont respectivement au moins partiellement pénétré dans au moins l'une des substances (ST), et
b) générer respectivement un taux de comptage (ZR1, ZR2, ZR3, ZR4) respectif ou une grandeur mesurée respective dépendant du taux de comptage uniquement sur la base de rayons gamma (GR) respectivement détectés dont l'énergie gamma (GE) respective est égale ou supérieure à une valeur de seuil d'énergie (ETh2), la valeur de seuil d'énergie (ETh2) étant au moins égale à 0,5 fois une valeur d'énergie Compton (CE) d'un intervalle Compton (CG) des rayons gamma (GR),
- dans lequel le procédé comporte l'étape consistant à : émettre respectivement des rayons gamma respectifs (GR) ayant une énergie gamma isotopique discrète (IGE) dans au moins l'une des substances (ST) au moyen d'une pluralité de sources de rayonnement (31, 32, 33, 34), la valeur d'énergie Compton (CE) étant inférieure à l'énergie gamma isotopique (IGE).

2. Procédé selon la revendication 1, dans lequel le procédé comporte l'étape consistant à :
c) déterminer le profil de densité (DP) sur la base des taux de comptage respectivement générés (ZR1, ZR2, ZR3, ZR4) ou des grandeurs mesurées respectivement générées.

3. Procédé selon l'une des revendications précédentes,
- dans lequel, lors de l'étape a), la détection consiste respectivement à : générer respectivement des impulsions de signal de détecteur (DI1, DI2, DI3, DI4) respectives au moyen des détecteurs (41, 42, 43, 44), des formes respectives, en particulier des amplitudes (IA1, IA2, IA3, IA4) et/ou des largeurs et/ou des produits des amplitudes et des largeurs, des impulsions de signal de détecteur respectivement générées (DI1, DI2, DI3, DI4) étant dépendantes des énergies gamma (GE) respectives des rayons gamma (GR) respectifs, et
- dans lequel l'étape b) consiste à : générer respectivement le taux de comptage (ZR1, ZR2, ZR3, ZR4) respectif ou la grandeur mesurée respective uniquement sur la base d'impulsions de signal de détection respectivement générées (DI1, DI2, DI3, DI4) dont les formes respectives sont égales ou supérieures à une valeur de seuil de forme (ATh2), la valeur de seuil de forme (ATh2) étant dépendante de la valeur de seuil d'énergie (ETh2).

4. Procédé selon l'une des revendications précédentes,
- dans lequel les détecteurs (41, 42, 43 44) présentent un bruit de détection ayant une valeur d'énergie de bruit (NE), la valeur de seuil d'énergie (ETh2) étant au moins égale à 2 fois la valeur d'énergie de bruit (NE), et/ou
- dans lequel la valeur de seuil d'énergie (ETh2) est au moins égale à la valeur d'énergie Compton (CE).

5. Procédé selon l'une des revendications précédentes,
- dans lequel les détecteurs (41, 42, 43, 44) sont disposés latéralement à l'extérieur par rapport à une paroi (22), en particulier ronde, du récipient (20), et/ou
- dans lequel les détecteurs (41, 42, 43, 44) sont disposés verticalement les uns au-dessus des autres.

6. Procédé selon l'une des revendications précédentes, notamment la revendication 5,
- dans lequel les sources de rayonnement (31, 32, 33, 34) sont disposées à l'intérieur du récipient (20).

7. Procédé selon l'une des revendications précédentes, notamment la revendication 6,
- dans lequel les détecteurs (41, 42, 43, 44) sont respectivement dirigés vers une source de rayonnement (31, 32, 33, 34) associée ou vers un point, notamment central, situé entre deux sources de rayonnement (31, 32, 33, 34) associées.

8. Procédé selon la revendication 7, notamment selon la revendication 5,
- dans lequel les détecteurs (41, 42, 43, 44) sont respectivement disposés à la même hauteur que la source de rayonnement (31, 32, 33, 34) associée ou à la même hauteur que le point situé entre les deux sources de rayonnement (31, 32, 33, 34) associées.

9. Procédé selon l'une des revendications précédentes, notamment selon l'une des revendications 6 à 8,
- dans lequel les détecteurs (41, 42, 43, 44) comportent respectivement un collimateur (51, 52, 53, 54), les collimateurs (51, 52, 53, 54) limitant respectivement un angle d'incidence (EW), notamment respectivement sur la ou les sources de rayonnement (31, 32, 33, 34) associées, et/ou
- dans lequel les sources de rayonnement (31, 32, 33, 34) comportent respectivement un collimateur (81, 82, 83, 84), les collimateurs (81, 82, 83, 84) limitant respectivement un angle d'incidence (AW), notamment respectivement sur le ou les détecteurs associés (41, 42, 43, 44) .

10. Procédé selon l'une des revendications précédentes,
- dans lequel les détecteurs (41, 42, 43, 44) comportent respectivement un scintillateur (61, 62, 63, 64) permettant de détecter des rayons gamma (GR) respectifs, les scintillateurs (61, 62, 63, 64) présentant une densité d'au moins 3 g/cm³, notamment d'au moins 5 g/cm³, et/ou d'au plus 20 g/cm³, notamment d'au plus 10 g/cm³, notamment de 7 g/cm3.

11. Procédé selon l'une des revendications précédentes,
- dans lequel les substances (ST) comportent, notamment sont, un gaz (GAS), une mousse (FOAM), une huile (OIL), une émulsion (EMULSION), de l'eau (WATER) et/ou du sable (SAND), et/ou dans lequel le récipient (20) est un séparateur huile/eau (21), et/ou
- dans lequel les substances comportent un gaz, un hydrocarbure et/ou un acide, et/ou dans lequel le récipient est un séparateur hydrocarbure-acide.

12. Dispositif (10) de mesure de taux de comptage (ZR1, ZR2, ZR3, ZR4) ou de grandeurs mesurées dépendant des taux de comptage permettant une détermination d'un profil de densité (DP) d'au moins deux substances (ST) de densités différentes (DE) disposées à l'intérieur d'un récipient (20) au moyen d'une pluralité de détecteurs (41, 42, 43, 44), notamment pour la mise en œuvre d'un procédé selon l'une des revendications précédentes, dans lequel le dispositif (10) comporte :
- la pluralité de détecteurs (41, 42, 43, 44), les détecteurs (41, 42, 43, 44) étant respectivement conçus pour détecter des rayons gamma (GR) respectifs ayant respectivement au moins partiellement traversé l'une des substances (ST), et
- une pluralité d'équipements de génération (71, 72, 73, 74), les équipements de génération (71, 72, 73, 74) étant respectivement conçus pour générer un taux de comptage (ZR1, ZR2, ZR3, ZR4) respectif ou une grandeur mesurée respective dépendant du taux de comptage, uniquement sur la base de rayons gamma (GR) respectivement détectés, dont l'énergie gamma (GE) respective est égale ou supérieure à une valeur de seuil d'énergie (ETh2), la valeur de seuil d'énergie (ETh2) étant au moins égale à 0,5 fois une valeur d'énergie Compton (CE) d'un intervalle Compton (CG) des rayons gamma (GR),
- dans lequel le dispositif (10) comporte : une pluralité de sources de rayonnement (31, 32, 33, 34), les sources de rayonnement (31, 32, 33, 34) étant respectivement conçues pour émettre des rayons gamma (GR) respectifs ayant une énergie gamma isotopique discrète (IGE) dans au moins l'une des substances (ST), la valeur d'énergie Compton (CE) étant inférieure à l'énergie gamma isotopique (IGE).

13. Dispositif (10) selon la revendication 12, dans lequel le dispositif (10) comporte :
- un équipement de détermination (80), l'équipement de détermination (80) étant conçu pour déterminer le profil de densité (DP) sur la base des taux de comptage (ZR1, ZR2, ZR3, ZR4) respectivement générés ou des grandeurs mesurées respectivement générées.

14. Dispositif (10) selon la revendication 12 ou 13, dans lequel le dispositif (10) comporte :
- le récipient (20).
